# EUROPEAN PATENT APPLICATION

(11) **EP 4 806 506 A1**
(43) Date of publication of application: **16.09.2026**
(21) Application number: 25185499.8
(22) Date of filing: 26.06.2025
(51) Int. Cl.: A61N 5/10, G21F 3/00, G21K 1/02, G21K 1/10

(54) **RADIATION SHIELDING STRUCTURE**

(30) Priority: 13.03.2025 TW 114109288
(71) Applicant: Heron Neutron Medical Corp., Zhubei City, Hsinchu County (TW)
(72) Inventor: CHEN, Wei-Lin, Zhubei City, Hsinchu County (TW); TSAI, Wen-Chyi, Zhubei City, Hsinchu County (TW)
(74) Representative: Patentship Patentanwaltsgesellschaft mbH

(57) **Abstract**

A radiation shielding structure (200) is provided. The radiation shielding structure (200) includes a filter module (210), a shielding module (220), and a structural wall (230). The filter module (210) has a first aperture (211) for a neutron beam to pass through. The shielding module (220) has a second aperture (221). The structural wall (230) has a third aperture (231) for output of the neutron beam. The shielding module (220) is configured in front of the structural wall (230) or directly behind the filter module (210). The shielding module (220) may be moved in a direction perpendicular to the direction of the neutron beam so that the shielding module (220) may be moved to an open state or a closed state.

## Description

### TECHNICAL FIELD OF THE INVENTION

The present invention relates to a radiation shielding structure. In particular, it is used to shield photons radiated into a treatment room due to neutron activation after Boron Neutron Capture Therapy (BNCT) irradiation is stopped.

### BACKGROUND OF THE INVENTION

Boron Neutron Capture Therapy (BNCT) is an innovative radiation therapy for cancer that uses boron-containing drugs (e.g., amino acids as boron carriers) that are designed to accumulate specifically in the tumor cells of the patient while being absorbed less by normal cells. When a patient is exposed to epithermal neutrons, the tumor cells where the boron-containing drug has accumulated will absorb the neutrons more readily than normal cells. When the neutrons interact with the boron-containing drug, a nuclear reaction occurs to produce lithium nuclei (⁷Li) and α particles. The generated α particles and lithium nuclei (⁷Li) atoms have a very short range (about 5-9 microns) to destroy tumor cells. This range is roughly equal to the diameter of a cell, which enables the energy to be focused on the inside of the tumor cells, causing minimal damage to the neighboring healthy tissues. Therefore, BNCT has the advantages of being highly selective for the tumor affected area, having low toxicity, having fewer treatments than traditional radiotherapy, imposing less burden on the human body, and having a faster recovery period.

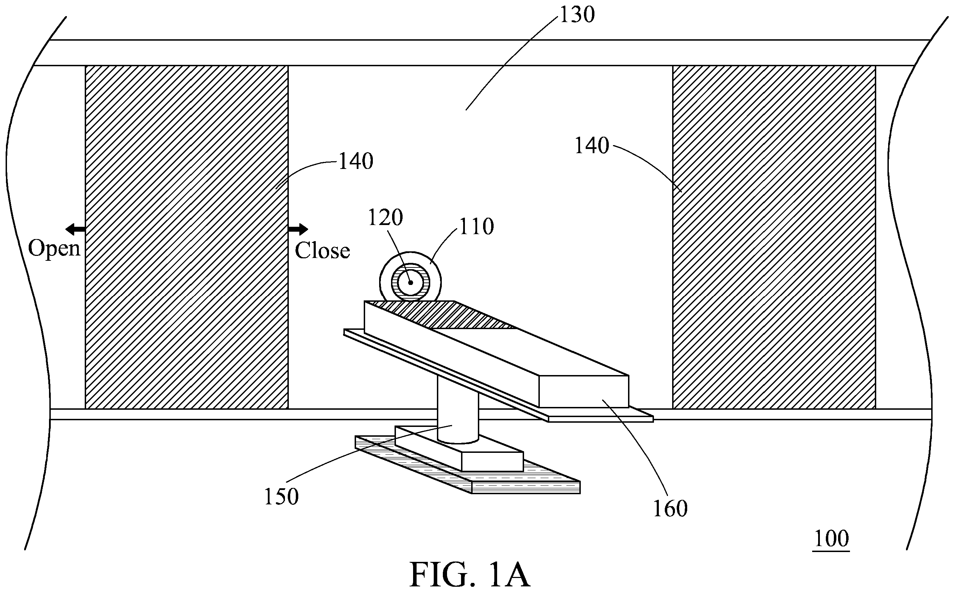

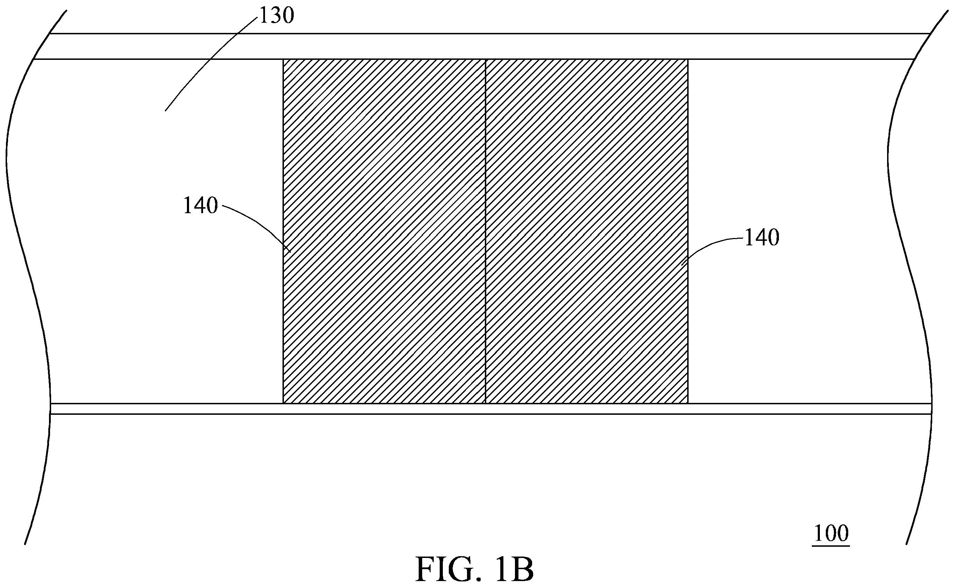

Referring to FIGS. 1A and 1B, these figures depict a prior art of BNCT treatment facility where a patient is treated. The treatment facility for BNCT typically includes a treatment room 100. The treatment room 100 further includes a medical bed 160, a robotic arm 150, a beam port 120, a Beam Shaping Assembling (BSA), a collimator 110, a neutron source (not shown), a structural wall 130, and a radiation shielding door 140. The BSA and the neutron source are located inside the structural wall 130, and the patient lies on the medical bed 160 in the treatment room 100. The medical bed 160 is connected to the robotic arm 150. When the treatment is started, the radiation shielding door 140 is opened as shown in FIG. 1A. The collimator 110 is installed at the beam port 120, and the robotic arm 150 controls the patient's affected part to move to the position of the beam port 120. The neutron source generates a neutron beam and irradiates the patient's affected part through the BSA located in the structural wall 130 and the collimator 110. After the treatment is completed, the neutron source stops generating the neutron beam. The collimator 110 is then removed, and the radiation shielding door 140 is closed as in FIG. 1B to isolate the radiation (e.g., Gamma ray) generated in the form of photons from the neutron-activated radioactive material, thereby reducing the dose of radiation that leaks from the beam port to the outside world.

However, deficiencies remain in the conventional configuration of the beam port 120, BSA, collimator 110, neutron source, radiation shielding door 140 aforementioned. Specifically, when treatment ends, the collimator 110 is still installed at the beam port 120 and must be removed before the shielding door 140 (outside structural wall 130) close, in other words, it means the door cannot be closed immediately after the treatment. The radiation shielding door 140 can only be closed after the photon dose has weakened to a certain level, and the treatment personnel enters the treatment room 100 to remove the collimator 110. For example, the BNCT treatment facilities at the Southern Tohoku BNCT Research Center in Japan and the Helsinki University Hospital Cancer Center in Finland all belong to this type of structure.

Furthermore, the need to wait for radiation levels to weaken means the patient in the treatment room 100 receives additional unnecessary radiation exposure after the treatment. This outcome partly offsets BNCT's advantages (low toxicity and fewer required treatments compared to conventional radiation treatments). Moreover, having to wait wastes valuable time in a medical setting, reducing facility efficiency and increasing cost. From the perspective of operation and management of long-term use, the accumulated delay time will reduce the medical facility utilization efficiency and increase the economic costs. Therefore, the radiation shielding structure of BNCT treatment in the current market is still in need of further improvement.

### BRIEF SUMMARY OF THE INVENTION

For the above reasons, a novel radiation shielding structure is provided in the present invention. The invention optimizes the radiation shielding approach in the treatment room, including aspects related to the collimator, structural wall, and the primary shielding barrier (achieved by the inventive shielding module), so that this shielding module can be closed at any time, whether or not the neutron source is active or residual radiation is present along the neutron beam path before/after treatment. With this improved radiation shielding structure, medical personnel can easily remove or replace the collimator as needed. Because the shielding module may be closed immediately after treatment, patients are not continuously exposed to high-dose radiation once treatment is completed. Thus, the advantages of the BNCT treatment technology (low toxicity, less burden on the body, and quicker recovery) can be better utilized. For the operation management, the utilization efficiency of the treatment equipment is also increased and the economic cost is reduced. In summary, the detailed technical solutions of the present invention are provided in the following sections.

A radiation shielding structure is provided in a first generalized embodiment of the present invention. The radiation shielding structure includes a filter module, a shielding module, and a structural wall. The filter module has a first aperture for a neutron beam to pass through. The shielding module has a second aperture. The structural wall has a third aperture for outputting of the neutron beam. The shielding module is configured in front of the structural wall. According to the embodiment of the present invention, the shielding module is movable in a direction perpendicular to the direction of the neutron beam to switch the shielding module between an open state and a closed state. In the embodiment, when the first aperture, the second aperture, and the third aperture are aligned, the radiation shielding structure is in an open state, and when the first aperture, the second aperture, and the third aperture are not aligned, the radiation shielding structure is in a closed state. In this way, when the radiation shielding structure is in the open state, the neutron beam can be outputted for treatment, and when the radiation shielding structure is in the closed state, the neutron beam and photons can be shielded. Therefore, the purpose of shielding radiation of the present invention can be achieved.

A radiation shielding structure is provided in a second generalized embodiment of the present invention. The radiation shielding structure includes a filter module, a shielding module, and a collimator. The filter module has a first aperture for a neutron beam to pass through. The shielding module has a second aperture. The collimator has a collimation aperture and is set on the shielding module. The collimation aperture is set on top of the shielding module and is aligned to the second aperture. The shielding module is behind the filter module. According to the embodiment, the shielding module is movable in a direction perpendicular to the direction of the neutron beam between an open state or a closed state. In the embodiment, when the first aperture, the second aperture, and the collimation aperture are aligned, the shielding module is in the open state, and when the first aperture, the second aperture, and the collimation aperture are not aligned, the shielding module is in the closed state. In this way, when the radiation shielding structure is in the open state, the neutron beam can be outputted for treatment, and when the radiation shielding structure is in the closed state, the neutron beam and photons can be shielded. Therefore, the purpose of shielding radiation of the present invention can be achieved.

### BRIEF DESCRIPTION OF THE DRAWINGS

The present invention can be more fully understood by reading the subsequent detailed description and examples with references made to the accompanying drawings. However, it should be understood that this is intended only as a reference for understanding the application of the present invention and not as a limitation of the invention to a particular embodiment.
FIG. 1A shows the configuration of the components (e.g., collimator, structural wall, radiation shielding door) of the treatment room in the prior art.
FIG. 1B shows the configuration of the radiation shielding door when closed in the treatment room in the prior art.
FIG. 2 shows the context of the treatment in the present invention, the direction in which the shielding module is opened and closed, and the associated configuration.
FIG. 3 shows the shielding module switching operation in an embodiment of the present invention in a stereoscopic view.
FIG. 4 shows the shielding module switching operation in another embodiment of the present invention in a stereoscopic view.
FIG. 5A shows the external structure of the collimator in the present invention.
FIG. 5B shows the external structure of the collimator in the present invention.
FIG. 6 shows the shielding module in the second generalized embodiment state with the shielding module in switching operation.

### DETAILED DESCRIPTION OF THE INVENTION

The present invention will be described in detail in terms of preferred embodiments and aspects so that the reader will thoroughly understand the manner in which these embodiments are practiced. However, the person skilled in the art should understand that the invention also can be practiced without these details. In addition, the present invention may be utilized and implemented by other specific embodiments. The details described herein may be applied to different needs, and various modifications or variations may be made. Therefore, the present invention will be described in terms of preferred embodiments and aspects; these descriptions serve to explain the structure of the present invention and are for the illustrative purposes only, and are not intended to limit the scope of the patent claims of the present invention. The terms used in the following descriptions will be interpreted in the broadest reasonable manner, and those skilled in the art may adjust the structure of the invention to meet the needs of the actual industry in accordance with the requirements of manufacturing or therapeutic applications, as described herein.

As used in the present invention, the terms such as "around", "approximately" or "substantially" shall normally mean within twenty percent, preferably within ten percent, and more preferably within five percent of a given value or range. The values given herein are approximate, meaning that the meaning of the terms "about", "approximately" or "substantially" may be inferred if not explicitly stated. In addition, the direction terms used in the present invention such as "front and back", "left and right", "up and down", etc., can be understood in conjunction with the description of the diagrams. Unless otherwise stated in the present invention, the meaning of the terms can be inferred after reading the contents of the description of the present invention. Finally, in order to simplify the drawings, some of the structures and components that are commonly used will be shown in the drawings in a simple schematic manner. And in order to facilitate viewing, the dimensions of the components in the drawings are not drawn according to the actual scale, which is described herein first.

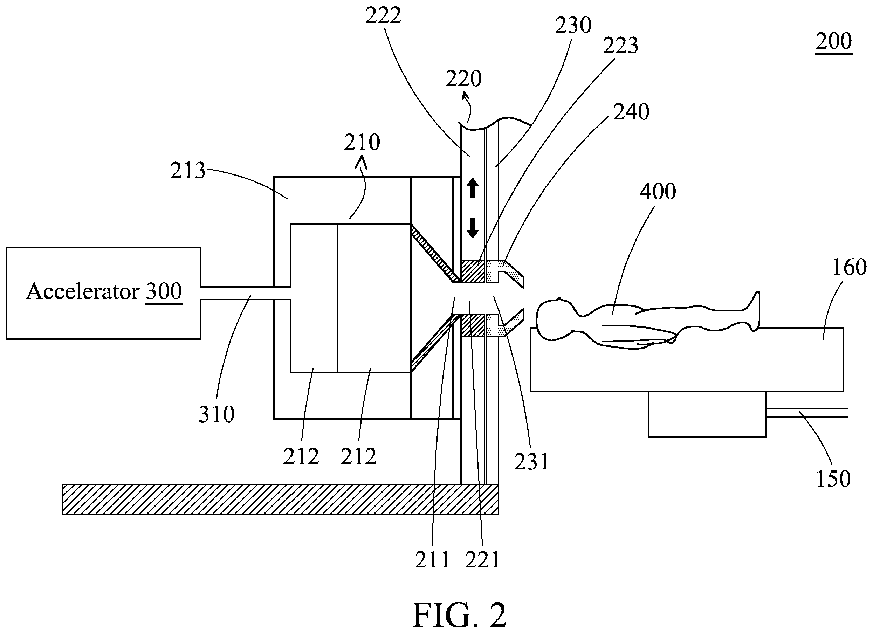

Referring to FIG. 2, the radiation shielding structure 200 of the present invention is applied to a radiotherapy system. The radiotherapy system includes an accelerator 300 and a beam channel 310. The beam channel 310 is connected to the accelerator 300 to provide a radiation source. The accelerator 300 can be, for example, a linear accelerator, cyclotron, or synchrotron radiation. The accelerator 300 interacts with components in the filter module 210 to generate the radiation beam. Depending on the embodiment of the system, the radiation source could be, but is not limited to, X-rays (e.g., high-energy X-rays), Gamma-rays (e.g., cobalt-60), a Proton beam (e.g., Proton Boron Fusion Therapy (PBFT)), or a Neutron beam (Boron Neutron Capture Therapy (BNCT)). In the present invention, the radiation is a neutron source for performing BNCT treatment.

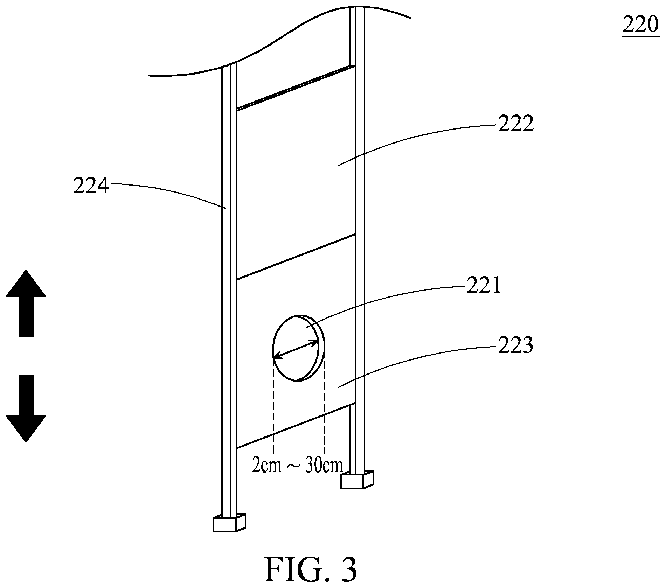

Referring to FIG. 2 and FIG. 3, a radiation shielding structure 200 according to a first generalized embodiment of the present invention is provided. The radiation shielding structure 200 includes a filter module 210, a shielding module 220, and a structural wall 230. The first aperture 211 is disposed on the filter module 210 for the neutron beam to pass through. The shielding module 220 is equipped with a second aperture 221. The structural wall 230 is equipped with a third aperture 231 for outputting of the neutron beam. The shielding module 220 is set in front of the structural wall 230. According to the first generalized embodiment of the present invention, the shielding module 220 is movable in a direction perpendicular to the direction of the neutron beam (e.g., up and down, left and right, clockwise and counterclockwise) to switch between an open state or a closed state. When the shielding module 220 is moved so that the first aperture 211, the second aperture 221, and the third aperture 231 are aligned, the shielding module 220 is in the open state. When the shielding module 220 is moved so that the first aperture 211, the second aperture 221, and the third aperture 231 are not aligned, the shielding module 220 is in the closed state. In this way, when the radiation shielding structure 200 is in the open state, the neutron beam is output for treatment, and when the radiation shielding structure 200 is in the closed state, the neutron beam and photons are shielded. According to one aspect of the present invention, since the shielding module 220 is configured in front of the structural wall 230, its movement will not affect the configuration of any components on the side of the structural wall 230 facing the patient 400 (i.e., in the medical space where the patient 400 is located), regardless of how the shielding module 220 moves. In particular, when the treatment is performed, the patient 400 is positioned very close to the structural wall 230(about 0.5-25cm away). This configuration allows medical staff to close the shielding module 220 after treatment without moving the patient 400, thereby reducing the patient's exposure time to high-dose radiation. Thus, the advantages of the BNCT treatment technology can be more optimally utilized.

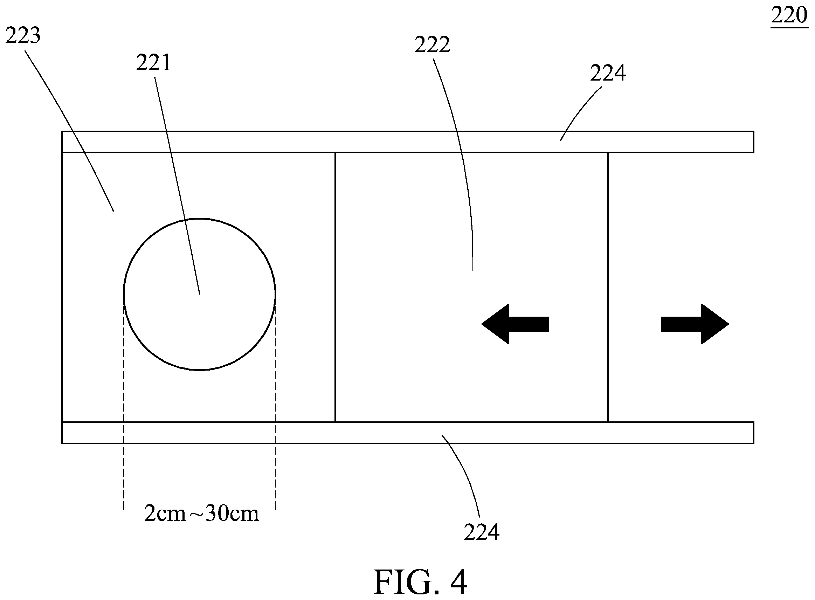

Referring to FIGS. 3A, 3B, 4A, and 4B, according to an embodiment of the present invention, the shielding module 220 has the appearance of a gate-blade structure. The gate-blade structure may be configured as a rectangle, polygon, trapezoid, ellipse, any shape, or a combination thereof according to the needs of the application. The gate-blade structure includes a first region 222 and a second region 223. The second aperture 221 is located in the second region 223, and the second aperture 221 penetrates the gate-blade structure of the shielding module 220 to allow neutron beams to pass through. The first region 222 is a radiation shielding material, which may be specifically selected from lead, silicon, boron, carbon, zinc, aluminum, nickel, tungsten, bismuth, copper, titanium, iron, or a combination thereof, and the second region is a neutron shielding material. In addition, in an embodiment of the present invention, the second aperture 221 is a circular hole with a diameter ranging from 2 centimeters to 30 centimeters. In another embodiment of the present invention, the second aperture 221 may also be selected from rectangular, polygonal, trapezoidal, elliptical, any shape, or a combination thereof. Further, in another embodiment of the present invention, the number and arrangement of the second aperture 221 may be configured according to the actual needs, such that the person skilled in the art may select the second aperture 221 according to the needs of the treatment (e.g., the scope of the tumor to be treated or the type of tumor).

Referring to FIGS. 3 and 4. According to an embodiment of the present invention, the radiation shielding structure 200 further includes at least one slide rail 224 located on the shielding module 220. Namely, the slide rail 224 is located on the outside of the gate-blade structure (e.g., the sides shown in FIGS. 3 to 4), such that the shielding module 220 can adjust the relative positions (e.g., up and down, left and right, or rotate) of the second aperture 221 to the first aperture 211 and the third aperture 231 in space by moving along the slide rail 224 to control the shielding module 220 to be in the open state or the closed state.

In an embodiment of the present invention, as shown in FIGS. 2 and 3, the slide rail 224 is set at the sides of the gate-blade structure and is perpendicular to the ground, such that the shielding module 220 may be moved in an upward and downward sliding manner. When the shielding module 220 moves upward to a predetermined open position, the shielding module 220 is in the open state such that neutron beams may pass through the second aperture 221. When the shielding module 220 is moved downward to a predetermined closed position, the shielding module 220 is in the closed state such that the radiation and/or neutron beams are blocked by the first region 222 and cannot pass through the second aperture 221. In another embodiment of the present invention, as shown in FIG. 4, the slide rails 224 are set on the sides of the gate-blade structure and are parallel to the ground, such that the shielding module 220 may be moved in a left-right sliding manner. When the shielding module 220 is moved to the left to a predetermined open position, the shielding module 220 is in the open state, such that the neutron beam may be passed through the second aperture 221. When the shielding module 220 is moved to the right to a predetermined closed position, the shielding module 220 is in the closed state, such that the radiation and/or the neutron beams are blocked by the first region 222 and cannot pass through the second aperture 221. It should be noted that in the above two embodiments, the description of the present invention only cites two examples of the shielding module 220 being able to achieve the open state and the closed state. However, a person skilled in the art should understand that, in addition to the upward and downward movement (as shown in FIG. 3) and left and right movement (as shown in FIG. 4), the movement can also be clockwise and counterclockwise rotation. The predetermined open position, the predetermined closed position, the relative position of the first region 222 and the second region 223, and the distance that the shielding module is moved may be modified by the needs of the application. The above embodiments are only for the purpose of illustration, and are not intended to limit the scope of the claim of the present invention, and are hereby described in particular.

According to an embodiment of the present invention, the filter module 210 is equipped with the first aperture 211 for the neutron beam to pass through. The accelerator 300 is used to generate the neutron beam with the other components of the filter module 210, and after the neutron beam passes through the filter module 210, the neutron beam with the required energy for medical treatment is generated. The filter module 210 may include an outer shell 213 and at least one filter layer 212. The neutron beam travels generally parallel to the ground and passes sequentially through the first aperture 211, the second aperture 221, and the third aperture 231.

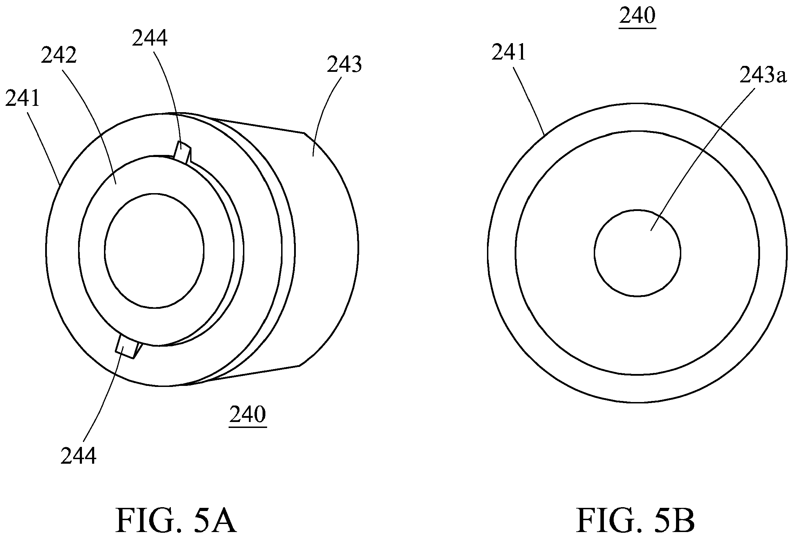

In FIGS. 2, 5A and 5B, according to an embodiment of the present invention, the radiation shielding structure 200 further includes a collimator 240. The collimator 240 further includes a base 241, a guidance section 242, a collimation section 243, and at least one fixing section 244, such that the collimator 240 is bonded to the structural wall 230 by the base 241 and encompasses the third aperture 231. The guidance section 242 guides the collimator 240 to slide in the corresponding fixed structure of the structural wall 230, and the fixing section 244 is finally fastened to the structural wall 230. When the shielding module 220 is in the open state, the neutron beam passes through the first aperture 211, the second aperture 221, the third aperture 231, and the collimating aperture 243a to carry out the radiation treatment for the patient 400. The collimator 240 thus adjusts the irradiation site and shape of the neutron beam's output.

In an embodiment of the present invention, the base 241 of the collimator 240 is in a flat shape, which further may be a pie shape. The guidance section 242 and the fixing section 244 are set on one side of the base 241 of the pie shape. The guidance section 242 is set circularly in the inside diameter of the base 241 and protrudes from one side of the plane of the base 241. The fixing section 244 is configured along the guidance section 242. The other side of the base 241 is equipped with the collimation section 243. In an embodiment, the collimation section 243 may be of a conical shape and extend from one side of the base 241 to the other side. The collimating aperture 243a is on the collimation section 243 and penetrates through the two sides of the collimator 240. The conical angle of the collimation section 243 and the size of the collimating aperture 243a may be set up according to the type of treatment. In an embodiment of the present invention, the materials of the base 241, the guidance section 242, the collimation section 243, and the fixing section 244 may be selected from a radiation shielding material, a neutron shielding material, or a combination thereof. The radiation shielding material may be selected from lead, silicon, boron, carbon, zinc, aluminum, nickel, tungsten, bismuth, copper, titanium, iron, or a combination thereof.

According to one aspect of the present invention, as shown in FIG. 2, since the shielding module 220 is set in front of the structural wall 230 and the collimator 240 in the radiation shielding structure 200, i.e., after the neutron beam is emitted from the accelerator 300, the neutron beam will pass through the accelerator 300, the beam channel 310, the filter module 210, the shielding module 220, the structural wall 230, and the collimator 240 sequentially. Therefore, the shielding module 220 will not affect one of the sides of the structural wall 230 near the patient 400 no matter how the shielding module 220 is moved, as described above. That is, no matter when the patient 400 is located in the medical space of the layout of any component, the medical personnel can select the appropriate specifications of the collimator 240 according to the needs of the treatment at any time as long as the shielding module 220 is in the closed state. For example, as shown in FIGS. 5A and 5B, the shape of the neutron beam irradiated to the target treatment site is close to a circle when the collimator aperture 243a is circular.

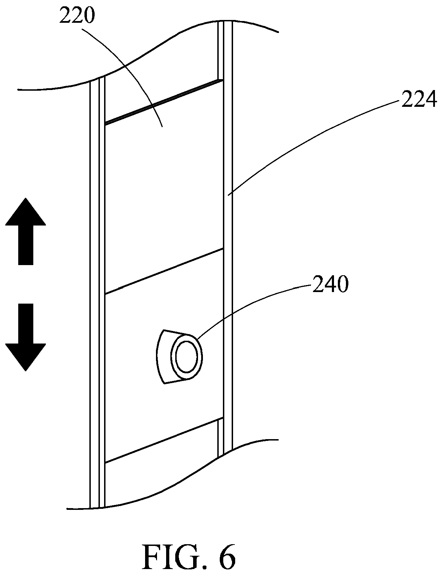

In FIG. 6, a radiation shielding structure 200 is provided in a second generalized embodiment. The radiation shielding structure 200 includes a filter module 210, a shielding module 220, and a collimator 240. The filter module 210 is equipped with a first aperture 211 for a neutron beam to pass through. The shielding module 220 is equipped with a second aperture 221. The collimator 240 is equipped with a collimation aperture 243a. The collimation aperture 243a is aligned to the second aperture 221. The shielding module 220 is set behind the filter module 210. According to an embodiment of the present invention, the shielding module 220 may be moved in a direction perpendicular to the direction of the neutron beam, such that the shielding module 220 may be moved to an open state or a closed state. When the first aperture 211, the second aperture 221, and the collimation aperture 243a are aligned, the shielding module 220 is in the open state; on the contrary, when the first aperture 211, the second aperture 221, and the collimation aperture 243a are not aligned, the shielding module 220 is in the closed state. Therefore, when the radiation shielding structure 200 is in the open state, the neutron beam could be outputted for treatment. When the radiation shielding structure 200 is in the closed state, the neutron beam and photons are shielded for the purpose of shielding the radiation in the present invention. According to the embodiment of the present invention, the movement of the shielding module 220 and the structure of the collimator 240 in the second generalized embodiment of the radiation shielding structure 200 are the same as those in the first generalized embodiment. Therefore, it will not be further elaborated herein. However, in the second generalized embodiment, the collimator 240 is mounted onto the second aperture 221 of the shielding module 220 by the base 241, and the guidance section 242 guides the collimator 240 to slide in the corresponding fixed structure of the shielding module 220. Finally, the collimator is fixed by the fixing section 244 on the shielding module 220, such that the neutron beam can pass through the first aperture 211, the second aperture 221 and the collimation aperture 243a in the open state of the shielding module 220 in order to carry out the radiation treatment on the patient 400. Therefore, the collimator 240 is able to adjust the site and the shape of the irradiation for the final output of the neutron beam. When the shielding module 220 is moved to the closed state, the neutron beam and photons are shielded for the purpose of shielding the radiation in the present invention. The medical bed 160 may be moved slightly by the robotic arm 150 according to the needs of the application while the shielding module 220 is being moved, such that the medical personnel can conveniently replace or disassemble the collimator 240 when the shielding module 220 is in the closed state to achieve the purpose of the present invention.

In summary, the radiation shielding structure 200 provided in the present invention improves upon conventional configuration that include a radiation shielding door, collimator, structural wall. This improvement is achieved by, for example, setting the shielding module 220 in front of the structural wall 230 or by having the shielding module 220 (in conjunction with other elements like the filter module) effectively replace a section of the structural wall. This ensures that the shielding module 220, serving as the primary movable radiation barrier, can be closed at any time. The improved radiation shielding structure can allow the medical personnel to easily dismantle and replace the collimator. The time of continuous exposure to high doses of radiation can be reduced for the patients. The efficiency of the use of therapeutic equipment can be increased and economic costs can be reduced for the operation management. Therefore, the present invention is not only highly industrially applicable but also features a novel structure and improved performance. It should be noted that the person skilled in this field can make various modifications after reading the present invention, but none of them is outside the scope of protection as intended in the scope of the patent claim.

## Claims

1. A radiation shielding structure (200), **characterized by**, comprising:
a filter module (210), having a first aperture (211);
a shielding module (220), having a second aperture (221); and
a structural wall (230), having a third aperture (231), wherein the shielding module (220) is configured in front of the structural wall (230);
wherein when the first aperture (211), the second aperture (221), and the third aperture (231) are aligned, the radiation shielding structure (200) is in an open state;
wherein when the first aperture (211), the second aperture (221), and the third aperture (231) are not aligned, the radiation shielding structure (200) is in a closed state.

2. The radiation shielding structure (200) as claimed in claim 1, wherein the shielding module (220) appears as a gate-blade structure, and the gate-blade structure is a rectangle, polygon, trapezoid, ellipse, or a combination thereof.

3. The radiation shielding structure (200) as claimed in claim 2, further comprising:
at least one slide rail (224), configured at sides of the gate-blade structure, and the shielding module (220) is opened and closed through sliding;
wherein the shielding module (220) is moved in a direction perpendicular to a direction of a neutron beam.

4. The radiation shielding structure (200) as claimed in claim 1, wherein the shielding module (220) comprises:
a first region (222); and
a second region (223), wherein the second aperture (221) is configured in the second region (223);
wherein the first region (222) is a radiation shielding material, and the second region (223) is a neutron shielding material.

5. The radiation shielding structure (200) as claimed in claim 4, wherein the radiation shielding material is lead, silicon, boron, carbon, zinc, aluminum, nickel, tungsten, bismuth, copper, titanium, iron, or a combination thereof.

6. The radiation shielding structure (200) as claimed in claim 1, wherein the second aperture (221) is a circular hole with a diameter ranging from 2 centimeters to 30 centimeters.

7. The radiation shielding structure (200) as claimed in claim 1, further comprising:
a collimator (240), wherein the collimator (240) comprises:
a base (241), being flat in shape;
a guidance section (242), configured circularly in the inside diameter of the base (241) and protruding from one side of a plane of the base (241);
at least one fixing section (244), configured along the guidance section (242); and
a collimation section (243), configured on another side of the base (241), and having a collimation aperture (243a) that penetrates through two sides of the collimator (240).

8. The radiation shielding structure (200) as claimed in claim 7, wherein the collimation section (243) is a conical shape.

9. The radiation shielding structure (200) as claimed in claim 7, wherein the collimator (240) is a radiation shielding material, a neutron shielding material, or a combination thereof.

10. A radiation shielding structure (200), **characterized by**, comprising:
a filter module (210), having a first aperture (211);
a shielding module (220), having a second aperture (221); and
a collimator (240), having a collimation aperture (243a), wherein the shielding module (220) is behind the filter module (210);
wherein when the first aperture (211), the second aperture (221), and the collimation aperture (243a) are aligned, the radiation shielding structure (200) is in an open state; and
wherein when the first aperture (211), the second aperture (221), and the collimation aperture (243a) are not aligned, the radiation shielding structure (200) is in a closed state.

11. The radiation shielding structure (200) as claimed in claim 10, wherein the shielding module (220) appears as a gate-blade structure, and the gate-blade structure is a rectangle, polygon, trapezoid, ellipse, or a combination thereof;
wherein the shielding module (220) comprises:
a first region (222); and
a second region (223), wherein the second aperture (221) is configured in the second region (223);
wherein the first region (222) is a radiation shielding material, and the second region (223) is a neutron shielding material.

12. The radiation shielding structure (200) as claimed in claim 11, further comprising:
at least one slide rail (224), configured at sides of the gate-blade structure, which allows the shielding module (220) to open and close through sliding;
wherein the shielding module (220) is moved in a direction perpendicular to a direction of a neutron beam.

13. The radiation shielding structure (200) as claimed in claim 10, wherein the second aperture (221) is a circular hole with a diameter ranging from 2 centimeters to 30 centimeters.

14. The radiation shielding structure (200) as claimed in claim 10, wherein the collimator (240) further comprises:
a base (241), being flat in shape;
a guidance section (242), configured circularly in the inside diameter of the base (241) and protruding from one side of a plane of the base (241);
at least one fixing section (244), configured along the guidance section (242); and
a collimation section (243), configured on another side of the base (241), wherein the collimation aperture (243a) penetrates through a top of the collimation section (243).

15. The radiation shielding structure (200) as claimed in claim 10, wherein the collimator (240) is a radiation shielding material, a neutron shielding material, or a combination thereof.

## Amended claims

### Amended claims in accordance with Rule 137(2) EPC.

1. A radiation shielding structure (200), comprising:
a filter module (210), having a first aperture (211);
a shielding module (220), having a second aperture (221);
a structural wall (230), having a third aperture (231), wherein the shielding module (220) is between the filter module (210) and the structural wall (230); and
a collimator (240), disposed on the structural wall (230);
wherein when the first aperture (211), the second aperture (221), and the third aperture (231) are aligned, the radiation shielding structure (200) is in an open state;
wherein in the open state a neutron beam is allowed to be outputted for treatment;
wherein when the first aperture (211), the second aperture (221), and the third aperture (231) are not aligned, the radiation shielding structure (200) is in a closed state;
wherein in the closed state the neutron beam is shielded.

2. The radiation shielding structure (200) as claimed in claim 1, wherein the shielding module (220) appears as a slidable plate structure, and the slidable plate structure is a rectangle, polygon, trapezoid, ellipse, or a combination thereof.

3. The radiation shielding structure (200) as claimed in claim 2, further comprising:
at least one slide rail (224), configured at sides of the slidable plate structure, and the shielding module (220) is opened and closed through sliding;
wherein the shielding module (220) is moved in a direction perpendicular to a direction of the neutron beam.

4. The radiation shielding structure (200) as claimed in claim 1, wherein the shielding module (220) comprises:
a first region (222); and
a second region (223), wherein the second aperture (221) is configured in the second region (223);
wherein the first region (222) is a radiation shielding material, and the second region (223) is a neutron shielding material.

5. The radiation shielding structure (200) as claimed in claim 4, wherein the radiation shielding material is lead, silicon, boron, carbon, zinc, aluminum, nickel, tungsten, bismuth, copper, titanium, iron, or a combination thereof.

6. The radiation shielding structure (200) as claimed in claim 1, wherein the second aperture (221) is a circular hole with a diameter ranging from 2 centimeters to 30 centimeters.

7. The radiation shielding structure (200) as claimed in claim 1,
wherein the collimator (240) comprises:
a base (241), being flat in shape;
a guidance section (242), configured circularly in the inside diameter of the base (241) and protruding from one side of a plane of the base (240);
at least one fixing section (240), configured along the guidance section (242); and
a collimation section (243), configured on another side of the base (241), and having a collimation aperture (243a) that penetrates through two sides of the collimator (240).

8. The radiation shielding structure (200) as claimed in claim 7, wherein the collimation section (243) is a conical shape.

9. The radiation shielding structure (200) as claimed in claim 7, wherein the collimator (240) is a radiation shielding material, a neutron shielding material, or a combination thereof.

10. A radiation shielding structure (200), comprising:
a filter module (210), having a first aperture (211);
a shielding module (220), having a second aperture (221);
a collimator (240), having a collimation aperture (243a), wherein the shielding module (220) is between the filter module (210) and the collimator (240); and
a structural wall (230), wherein the collimator (240) is disposed on the structural wall (230);
wherein when the first aperture (211), the second aperture (220), and the collimation aperture (243a) are aligned, the radiation shielding structure (200) is in an open state;
wherein in the open state a neutron beam is allowed to be outputted for treatment;
wherein when the first aperture (211), the second aperture (221), and the collimation aperture (243a) are not aligned, the radiation shielding structure (200) is in a closed state;
wherein in the closed state the neutron beam is shielded.

11. The radiation shielding structure (200) as claimed in claim 10, wherein the shielding module (220) appears as a slidable plate structure, and the slidable plate structure is a rectangle, polygon, trapezoid, ellipse, or a combination thereof;
wherein the shielding module (220) comprises:
a first region (222); and
a second region (223), wherein the second aperture (221) is configured in the second region (223);
wherein the first region (212) is a radiation shielding material, and the second region (223) is a neutron shielding material.

12. The radiation shielding structure (200) as claimed in claim 11, further comprising:
at least one slide rail (224), configured at sides of the slidable plate structure, which allows the shielding module (220) to open and close through sliding;
wherein the shielding module (220) is moved in a direction perpendicular to a direction of the neutron beam.

13. The radiation shielding structure (200) as claimed in claim 10, wherein the second aperture (221) is a circular hole with a diameter ranging from 2 centimeters to 30 centimeters.

14. The radiation shielding structure (200) as claimed in claim 10, wherein the collimator (240) further comprises:
a base (241), being flat in shape;
a guidance section (242), configured circularly in the inside diameter of the base (241) and protruding from one side of a plane of the base (241);
at least one fixing section (244), configured along the guidance section (242); and
a collimation section (243), configured on another side of the base (240), wherein the collimation aperture (243a) penetrates through a top of the collimation section (243).

15. The radiation shielding structure (200) as claimed in claim 10, wherein the collimator (240) is a radiation shielding material, a neutron shielding material, or a combination thereof.
